## Europäisches Patentamt

## European Patent Office

## Office européen des brevets

(19)

(11) Publication number: **0 024 566**
**B1**

(12)

# EUROPEAN PATENT SPECIFICATION

(45) Date of publication of patent specification: **22.06.83**

(51) Int. Cl.³: **G 01 N 33/20**

(21) Application number: **80104512.1**

(22) Date of filing: **30.07.80**

(54) **An apparatus for the analysis of oxygen, nitrogen and hydrogen contained in metals.**

(30) Priority: **15.08.79 JP 104319/79**

(43) Date of publication of application:
**11.03.81 Bulletin 81/10**

(45) Publication of the grant of the patent:
**22.06.83 Bulletin 83/25**

(84) Designated Contracting States:
**DE FR GB IT SE**

(56) References cited:
**DE - A - 1 939 403**
**DE - C - 1 079 352**
**GB - A - 1 198 395**
**US - A - 2 932 558**
**US - A - 3 427 863**

(73) Proprietor: **HORIBA, LTD.**
**2 Miyanohigashi-machi Kissyoin**
**Minami-ku Kyoto (JP)**

(72) Inventor: **Mikasa, Hajime**
**1158-3, Katsube-cho**
**Moriyama-city Siga-ken (JP)**
Inventor: **Tsuji, Katsuya**
**3-3,3 Chome, Mizuki-dori**
**Hyogo-ku, Kobe-city Hyogo-ken (JP)**
Inventor: **Ono, Hirofumi**
**5-9, 1-Chome Onoasahi Siga-cho**
**Siga-gun Siga-ken (JP)**

(74) Representative: **Patentanwälte TER MEER -**
**MÜLLER - STEINMEISTER**
**Triftstrasse 4**
**D-8000 München 22 (DE)**

Courier Press, Leamington Spa, England

## An apparatus for the analysis of oxygen, nitrogen and hydrogen contained in metals

The present invention relates to an apparatus for the analysis of oxygen (hereinafter referred to as "O"), nitrogen (hereinafter referred to as "N") and hydrogen (hereinafter referred to as "H") contained in metals.

The analysis of O, N, H contained in metals has been done by suitably treating gaseous components such as carbon monoxide (CO), gaseous nitrogen ($N_2$) and gaseous hydrogen ($H_2$) generated by heating the sample containing O, N and H respectively in a graphite crucible to a temperature of about 3000°C with a carrier gas such as an inert gas and then detecting CO, $N_2$ and $H_2$ respectively by means of a thermal conductivity detector (TCD). However, this method comprises a two-stage determination, wherein the determination of O and N and the determination of H must be done separately, using helium (He) as carrier gas for the determination of O and N, and argon (Ar) as carrier gas for the determination of H. This two-stage method is necessary because of the fundamental reason that He is similar to $H_2$ and Ar is similar to $CO_2$ as well as to $N_2$ with respect to their thermal conductivities at the same temperature when a thermal conductivity detector (TCD) is used.

In practice, the determination of O as well as N and H has been done as follows.

(I) The measurement of O and N using He as carrier gas.

CO, $N_2$ and $H_2$ are generated by heating the sample in a graphite crucible to a high temperature. Then, $H_2$ and CO are oxidized to water vapour ($H_2O$) and carbon dioxide ($CO_2$), respectively by contacting them with an oxidizing agent such as cupric oxide heated to a temperature of about 400°C. The $H_2O$ thus obtained is removed by means of a dehydrating agent such as magnesium perchlorate and the gaseous mixture of $N_2$ and $CO_2$ is separated to $N_2$ and $CO_2$ by passing the mixture through a gas chromatographic column (filled with silica gel or the like), whereafter $N_2$ and $CO_2$ are detected by means of a thermal conductivity detector. The reason for using an oxidizing agent being, that it is difficult to quickly and completely separate the three components (CO, $N_2$ and $H_2$) by means of a gas chromatographic column. The reason for the use of a dehydrating agent is than $H_2O$ degrades the gas chromatographic column used for separating $N_2$ and $CO_2$.

(II) The measurement of H using Ar as carrier gas.

CO, $N_2$ and $H_2$ are generated from the sample as disclosed above in (I). Then, CO is oxidized to $CO_2$ by contacting the gaseous mixture obtained with a normal temperature oxidizing agent such as sodium periodate which does not oxidize $H_2$ but oxidizes CO. The $CO_2$ thus obtained is removed by an absorbing agent such as soda-asbestos and the mixture comprising $N_2$ and $H_2$ is introduced into a gas chromatographic column to separate $N_2$ from $H_2$ whereafter $H_2$ is detected by means of a thermal conductivity detector. The reason for removing $CO_2$ by means of an absorbing agent is that $CO_2$ degrades the gas chromatographic column used for separating $N_2$ and $H_2$.

As can be seen, it is necessary to use separate samples for the determination of O and N and for the determination of H during the analysis of O, N and H contained in metals. This means that it is necessary to use different carrier gases, agents, gas chromatographic columns or the like properly in accordance to what components are to be analysed. This method requires furthermore to repeat twice the preliminary operations of annealing the empty graphite crucible and purging the system which as a result slows down and complicates the analysis. In addition to that, the conventional method has further defects such as the complication of the apparatus, the costs and the like.

US—A—3 427 863 describes a method and an apparatus for the direct determination of gases in samples, such as nitrogen and oxygen in steel samples, which comprise heating the sample in a stream of carrier gas, such as helium, to release the gases to be determined and entrain them in the stream, trapping the gases out of the stream in a cold trap, disconnecting the cold trap from that stream and connecting it in a second stream of carrier gas flowing through a measuring cell, such as a thermal conductivity cell, and heating the trap to release the trapped gases therefrom. This method and apparatus obviously are complicated and do not allow an easy analysis of oxygen, nitrogen and hydrogen contained in metals.

GB—A—1 198 395 discloses a method and an apparatus for the determination of hydrogen in steel, comprising continuously feeding the primary stream of carrier gas, i.e. argon, and continuously dividing the primary stream into a first and a second secondary stream, the first stream flowing through a measuring side of a thermal conductivity cell and the seocnd stream flowing through a reference side of the thermal conductivity cell and then through an extraction chamber containing a sample of steel, which method contains a flushing step, in which the second stream of carrier gas after flowing through the extraction chamber flows through a sampling tube, a withdrawing step, in which the sample is heated to extract gases therefrom and these gases are collected into a container, a sampling step, wherein the gases filling the container are conveyed into the sampling tube and a measuring step, in which the gases in the sampling tube are flushed out with the first stream of carrier gas and fed through a chromatographic column and then through the measuring side of the thermal conductivity cell. This method and apparatus as well is very complicated and does not allow for the analysis of oxygen and nitrogen at the same time.

DE—A—1 939 403 discloses a method and an apparatus for the gas chromatographic analysis of gases, such as hydrogen, nitrogen and carbon monoxide in metals using a chromatographic column and argon as carrier gas. The apparatus disclosed comprises an electric arc for extracting the gases from the sample, and complicated controlling means and electronic devices for controlling the gas flow and measuring the gases.

It is the object of the present invention to solve the above noted problems of the prior art methods and to provide an apparatus for easily and speedily analyzing the three components O, N and H contained in metals, so that the concentration of $H_2$ is determined from the measurement value of the concentration of $H_2O$ and consequently the concentration of O, N and H may be measured by using only helium (He) as a carrier gas.

It has now been found that even though the thermal conductivity of $H_2O$ is similar to that of $CO_2$ and $N_2$, a sharp peak can be obtained for $H_2O$ by back-flushing a gas chromatographic column for separating $H_2O$ from $N_2$ and from $CO_2$. This allows the analysis of three components (O, N, H) contained in a single sample by only one measurement operation using only He as a carrier gas, even though the elution time of $H_2O$ is prolonged somewhat in comparison to that of $N_2$ and $CO_2$. The subject matter of the invention therefore comprises the apparatus according to claim 1. The subclaims are directed to preferred embodiments of this apparatus.

The apparatus for the analysis of oxygen, nitrogen and hydrogen contained in metals using a carrier gas, comprising a graphite crucible (1) for receiving the sample to be analyzed, a heating furnace (2) for heating said crucible, a first gas chromatographic column (5) and at least one thermal conductivity detector (TCD, TCD$_1$, TCD$_2$) for detecting $CO_2$, $N_2$ or $H_2O$ according to the invention is characterized by a second gas chromatographic column (6) for separating $CO_2$ from $N_2$, a passage changing-over valve (4) for back-flushing said first gas chromatographic column (5), an oxidizing device (3) for oxidizing any CO and $H_2$ contained in the gaseous mixture of CO, $N_2$ and $H_2$ generated from the sample to $CO_2$ and $H_2O$ respectively and by using only helium as carrier gas.

The apparatus according to the present invention can be used to determine the three components O, N and H by only one measurement operation using a single carrier gas (He) and a single sample in that the concentration of $H_2$ can be determined from the measurement value of the concentration $H_2O$, which is produced by oxidizing $H_2$ generated from the sample by means of the oxidizing device, and consequently allows a fast analysis and a simple construction of the apparatus.

The elimination of a dehydrating agent, an absorbing agent for $CO_2$ and the like leads to a reduction of dead-space in the passages of the apparatus, to an improvement of maintenance, and to a prevention of the second gas chromatographic column from degrading for a long term owing to the condition that $H_2O$ does not enter into said second gas chromatographic column, and the like.

In the following the invention is described in detail with reference to the drawings, which illustrate several preferred embodiments.

Fig. 1 shows the construction of one embodiment of the apparatus;

Fig. 2(A) and Fig. 2(B) show the flow diagram in case when the passage changing-over valve was changed to the state expressed by the solid lines and the state expressed by the broken lines in Fig. 1, respectively; and

Fig. 3 shows the concentration of another embodiment of the apparatus according to the invention.

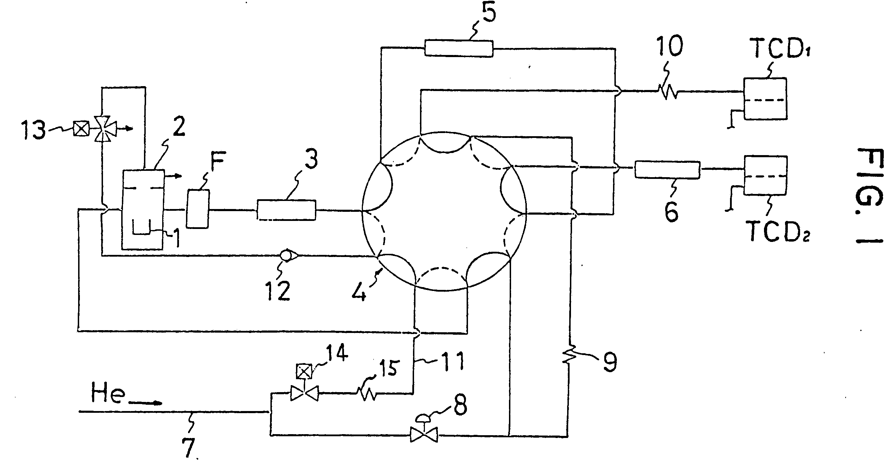

Fig. 1 shows a first preferred embodiment of the analyzing apparatus according to the present invention in which 1 is a graphite crucible for receiving the sample, 2 being a heating furnace for extracting gases, F being a filter, 3 being an oxidizing device containing an oxidizing agent such as cupric oxide, 4 being a passage changing over valve for back-flushing, 5 being a first gas chromatographic column for separating $H_2O$ from $CO_2$ and $N_2$, being filled with porous polymeric filler having a longer elution time for $H_2O$ than for $CO_2$ and $N_2$, 6 being a second gas chromatographic column for separating $CO_2$ from $N_2$, being filled with silica gel as filler, TCD$_1$ being a thermal conductivity detector for detecting $H_2O$, TCD$_2$ being a thermal conductivity detector for detecting $CO_2$ and $N_2$, 7 being a carrier gas (He being used) supplying line, 8 being a pressure regulator, 9, 10 being pneumatic resistances (for example capillary tubes), which as a rule have the same pneumatic resistances as said first gas chromatographic column 5 and said second gas chromatographic column 6, respectively (that it to say the pneumatic resistance 9 corresponds to the pneumatic resistance of said first gas chromatographic column 5 and the pneumatic resistance 10 corresponds to the pneumatic resistance of said second gas chromatographic column 6), wherein the pneumatic resistance 10 may be reduced so as to increase the flux in the back-flushing line in order to promote back-flushing of $H_2O$. Reference number 11 stands for a purging channel of said heating furnace 2 branched out from said carrier gas supplying line 7, 12 being a check valve and 13 being a three-way valve, 14 being an electromagnetic valve and 15 being a pneumatic resistance.

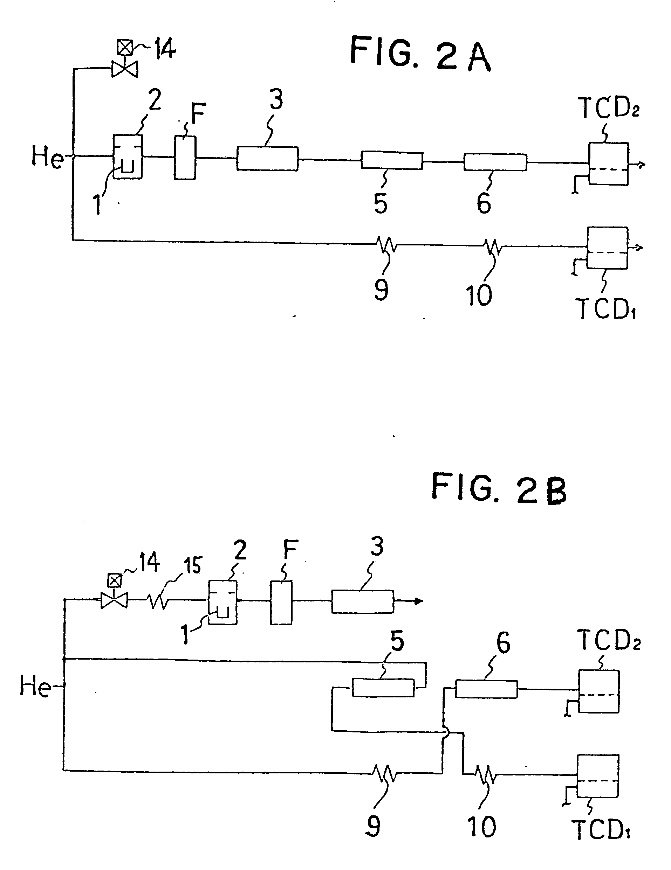

The method of analyzing using the apparatus of the invention shall now be described in detail, referring to Fig. 2(A) and Fig. 2(B). Fig. 2(A) shows the flow diagram in case when said passage changing-over valve 4 was changed over to the state expressed by the solid lines, whereas Fig. 2(B) represents the state expressed by the broken lines in Fig. 1.

At first said graphite crucible 1 is placed in said heating furnace 2 and then said heating furnace 2 is electrified to heat said graphite crucible 1 keeping the passage changing-over valve 4 in the state expressed by the broken lines in Fig. 1 (empty baking).

Then the sample is put into said graphite crucible 1 and the valve 4 is put back to the state expressed by the solid lines in Fig. 1, which is the stand-by state. In this state, said graphite crucible 1 has a normal-temperature.

Then said heating furnace is electrified to heat said graphite crucible suddenly up to about 3000°C, whereby O, N and H contained in said sample are subjected to a thermal decomposition generating CO, $N_2$ and $H_2$.

As shown in Fig. 2(A), said gases are transferred into said oxidizer 3 by said carrier gas He wherein CO and $H_2$ are transformed to $CO_2$ and $H_2O$, respectively. The gaseous mixture thus obtained consisting of $CO_2$, $N_2$ and $H_2O$ is introduced into said first gas chromatographic column 5, thereby separating $N_2$ and $CO_2$ from $H_2O$.

At the point when $N_2$ and $CO_2$ finish to eluate from said gas chromatographic column 5, the passages are changed over to the state expressed by the broken lines in Fig. 1 by means of said passage changing-over valve 4. The component that remains still in said first gas chromatographic column 5 is back-flushed and the peak of $H_2O$ eluated in the opposite direction is detected by the thermal conductivity detector $TCD_1$ (see Fig. 2(B)). As described above, the sensitivity is not enough when $H_2$ is detected by the thermal conductivity detector $TCD_1$ when using He as carrier gas. On the other hand the sensitivity is high when $H_2$ is transformed to $H_2O$ and $H_2O$ thus obtained is detected by the thermal conductivity detector $TCD_1$. The sensitivity in this case is similarly high as the sensitivity for the measurement of $N_2$ and $CO_2$. Although it is difficult to measure all of $N_2$, $CO_2$ and $H_2O$ by means of a single column because the adsorption of $H_2O$ is remarkable, the sharp peak of $H_2O$ can be obtained by using both said first column for separating $H_2O$ from $N_2$ and $CO_2$ and said second column for separating $N_2$ from $CO_2$ and by back-flushing said first column.

On the other hand, simultaneously with the detection of $H_2O$ the gaseous mixture of $N_2$ and $CO_2$ eluated out from said first column 5 is introduced into said second column 6 wherein $N_2$ is separated from $CO_2$ and each peak is detected by the thermal conductivity detector $TCD_2$.

Thus the components O, N and H contained in the single sample can be simultaneously determined by only one measuring operation using He as a carrier gas.

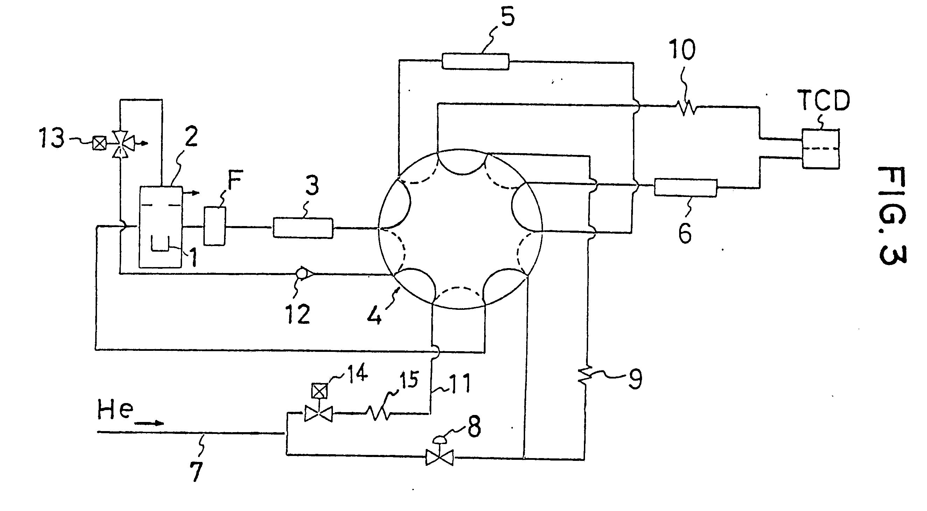

As shown in Fig. 2(B), when back-flushing is carried out under the condition that the passage changing over valve 4 is changed over to the state expressed by the broken lines in Fig. 1, the operator can purge and prepare the apparatus for the next measurement by passing the carrier gas (He) in the sequence of said heating furnace 2, said filter F, said oxidizer 3 and the atmosphere. Fig. 1 and Fig. 2(A), (B) relate to the embodiment of the invention comprising two colums 5, 6, double passages and two thermal conductivity detectors $TCD_1$ and $TCD_2$. According to another embodiment of the invention the analysis can be done by means of only one thermal conductivity detector TCD, as shown in Fig. 3. But, in the case shown in Fig. 3, $CO_2$ and $N_2$ are detected by means of the thermal conductivity detector TCD in turn (at this time $H_2O$ remains in said first column 5) whereafter said $H_2O$ remained in said first column 5 is back-flushed.

Furthermore, if $H_2O$ is back-flushed at the time when the $CO_2$ and $N_2$ are finished eluating from the first colum 5, it is necessary to prevent $CO_2$, $N_2$ and $H_2O$ from simultaneously arriving at the TCD. This is done by lengthening the second column 6 to delay the elution of $CO_2$ and $N_2$.

## Claims

1. An apparatus for the analysis of oxygen, nitrogen and hydrogen contained in metals using a carrier gas, comprising a graphite crucible (1) for receiving the sample to be analysed, a heating furnace (2) for heating said crucible, a first gas chromatographic column (5) and at least one thermal conductivity detector (TCD, $TCD_1$, $TCD_2$) for detecting $CO_2$, $N_2$ or $H_2O$ characterized by a second gas chromatographic column (6) for separating $CO_2$ from $N_2$, a passage changing-over valve (4) for back-flushing said first gas chromatographic column (5), an oxidizing device (3) for oxidizing any CO and $H_2$ contained in the gaseous mixture of CO, $N_2$ and $H_2$ generated from the sample to $CO_2$ and $H_2O$ respectively and by using only helium as carrier gas.

2. The apparatus according to claim 1, characterized in that said first gas chromatographic column (5) contains a filler having a longer elution time for $H_2O$ than for $CO_2$ and $N_2$.

3. The apparatus according to claim 1, characterized in that a channel (11) for purging said heating furnace (2) is branched from a carrier gas supplying line (7) through said passage changing-over valve (4) to said heating furnace (2).

4. The apparatus according to claim 1, characterized by two thermal conductivity detectors ($TCD_1$, $TCD_2$), one of which ($TCD_1$) is a detector for detecting $H_2O$ and the other ($TCD_2$) is a detector for detecting $N_2$ and $CO_2$.

5. The apparatus according to claim 1, characterized by only one thermal conductivity detector (TCD) to detect $N_2$, $CO_2$ and $H_2O$ with a time lag.

**Revendications**

1. Appareil pour l'analyse de l'oxygène, de l'azote et de l'hydrogène contenus dans des métaux, utilisant un gaz vecteur, comprenant un creuset en graphite (1) pour recevoir l'échantillon à analyser, un four thermique (2) pour le chauffage dudit creuset, une première colonne de chromatographie de gaz (5) et au moins un détecteur de conductivité thermique (TCD, $TCD_1$, $TCD_2$) pour détecter $CO_2$, $N_2$ ou $H_2O$, caractérisé en ce qu'il comporte une seconde colonne de chromatographie de gaz (6) pour séparer $CO_2$ de $N_2$, une vanne à plusieurs voies (4) pour inversion de ladite première colonne de chromatographie de gaz (5), un dispositif oxydant (3) pour oxyder le CO et $H_2$, contenus dans le mélange gazeux de CO, $N_2$ et $H_2$ engendrés à partir de l'échantillon, en $CO_2$ et $H_2O$ respectivement et que l'on utilise seulement l'hélium comme gaz vecteur.

2. Appareil selon la revendication 1, caractérisé par le fait que ladite première colonne de chromatographie de gaz contient un remplissage ayant un temps d'élution plus long pour $H_2O$ que pour $CO_2$ et $N_2$.

3. Appareil selon la revendication 1, caractérisé par le fait qu'un conduit (11) pour purger ledit four thermique (2) est branché de la ligne d'alimentation du gaz vecteur (7) par l'intermédiaire de ladite vanne à plusieurs voies (4) audit four thermique (2).

4. Appareil selon la revendication 1, caractérisé par le fait qu'il comporte deux détecteurs de conductivité thermique ($TCD_1$, $TCD_2$), dont l'un ($TCD_1$) est un détecteur pour détecter $H_2O$ et l'autre ($TCD_2$) est un détecteur pour détecter $N_2$ et $CO_2$.

5. Appareil selon la revendication 1, caractérisé par le fait qu'il comporte seulement un détecteur de conductivité thermique (TCD) pour

détecter $N_2$, $CO_2$ et $H_2O$ avec un temps de retard.

**Patentansprüche**

1. Vorrichtung zur Analyse von in Metallen enthaltenem Sauerstoff, Stickstoff und Wasserstoff unter Verwendung eines Trägergases, mit einem Graphittiegel (1) zur Aufnahme der zu analysierenden Probe, einem Heizofen (2) zum Aufheizen des Tiegels, einer ersten Gaschromatrographiesäule (5) und mindestens einem Wärmeleitfähigkeitsdetektor (TCD, $TCD_1$, $TCD_2$) zum Nachweis von $CO_2$, $N_2$ oder $H_2O$, gekennzeichnet durch eine zweite Gaschromatographiesäule (6) zur Trennung von $CO_2$ von $N_2$, ein Leitungsumschaltventil (4) zum Rückspülen der ersten Gaschromatographiesäule (5), eine Oxidationseinrichtung (3) zum Oxidieren von irgendwelchem CO und $H_2$ in der gasförmigen Mischung aus CO, $N_2$ und $H_2$, die aus der Probe freigesetzt worden ist, zu $CO_2$ bzw. $H_2O$ und durch die ausschließliche Verwendung von Helium als Trägergas.

2. Vorrichtung nach Anspruch 1, dadurch gekennzeichnet, daß die erste Gaschromatographiesäule (5) einem Füllstoff enthält der eine längere Elutionszeit für $H_2O$ als für $CO_2$ und $N_2$ aufweist.

3. Vorrichtung nach Anspruch 1, dadurch gekennzeichnet, daß ein Kanal (11) zum Spülen der Heizeinrichtung (2) von einer Trägergaszuführungsleitung (7) durch das Leitungsumschaltventil (4) zu dem Heizofen (2) abgezweigt ist.

4. Vorrichtung nach Anspruch 1, gekennzeichnet durch zwei Wärmeleitfähigkeitsdetektoren ($TCD_1$, $TCD_2$), von denen einer ($TCD_1$) ein Detektor zum Nachweis von $H_2O$ und der andere ($TCD_2$) ein Detektor zum Nachweis von $N_2$ und $CO_2$ darstellen.

5. Vorrichtung nach Anspruch 1, gekennzeichnet durch lediglich einen Wärmeleitfähigkeitsdetektor (TCD) zum zeitverzögerten Nachweis von $N_2$, $CO_2$ und $H_2O$.

FIG. 1

# FIG. 2 A

# FIG. 2 B

FIG. 3